(19) Europäisches Patentamt
European Patent Office
Office européen des brevets

(11) Veröffentlichungsnummer: **0 231 511 B1**

# (12) EUROPÄISCHE PATENTSCHRIFT

(45) Veröffentlichungstag der Patentschrift: **10.04.91**

(51) Int. Cl.[5]: **C07D 491/048**, A61K 31/44, //(C07D491/048,307:00,221:00)

(21) Anmeldenummer: **86117972.9**

(22) Anmeldetag: **23.12.86**

Die Akte enthält technische Angaben, die nach dem Eingang der Anmeldung eingereicht wurden und die nicht in dieser Patentschrift enthalten sind.

## (54) A-Aminoaryldihydropyridinlactone, Verfahren zu ihrer Herstellung und ihre Verwendung in Arzneimitteln.

(30) Priorität: **11.01.86 DE 3600596**

(43) Veröffentlichungstag der Anmeldung: **12.08.87 Patentblatt 87/33**

(45) Bekanntmachung des Hinweises auf die Patenterteilung: **10.04.91 Patentblatt 91/15**

(84) Benannte Vertragsstaaten: **AT BE CH DE ES FR GB IT LI NL SE**

(56) Entgegenhaltungen:
**EP-A- 0 111 453**
**DE-A- 3 130 041**
**DE-A- 3 410 645**
**US-A- 4 284 634**

(73) Patentinhaber: **BAYER AG**
**W-5090 Leverkusen 1 Bayerwerk(DE)**

(72) Erfinder: **Goldmann, Siegfried, Dr.**
**Am Osterholz 91**
**W-5600 Wuppertal 11(DE)**
Erfinder: **Schramm, Matthias, Dr.**
**Paffrather Strasse 38**
**W-5000 Köln 80(DE)**
Erfinder: **Gross, Rainer, Prof. Dr.**
**Platzhofstrasse 23**
**W-5600 Wuppertal 1(DE)**
Erfinder: **Thomas, Günther, Dr. c/o Bayer Italia S.p.A.**
**Rep. Pharmacologia Via delle Groane 126**
**I-20024 Garbagnate Mailand(IT)**
Erfinder: **Bechem, Martin, Dr.**
**Obere Bergerheide 4**
**W-5600 Wuppertal 1(DE)**
Erfinder: **Kayser, Michael, Dr.**
**Fleyerstrasse 231**
**W-5800 Hagen(DE)**

**EP 0 231 511 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 4-Aminoaryldihydropyrdinlactone, Verfahren zu ihrer Herstellung sowie ihre Verwendung in Arzneimitteln, insbesondere in Kreislaufbeeinflussenden Arzneimitteln.

Aus De-A 3 130 041, DE-A-3 410 645 und US 4 284 634 sind bereits Dihydropyridinlactone als pharmazeutische Wirkstoffe bekannt.

Die vorliegende Erfindung betrifft neue 4-Aminoaryldihydropyridine der allgemeinen Formel (I)

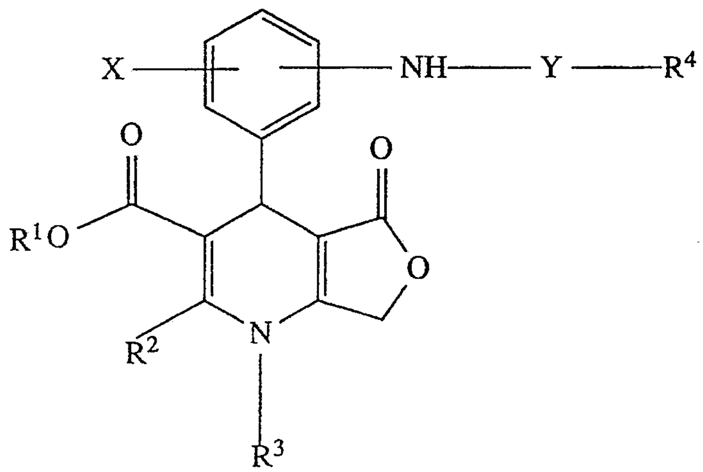

(I)

in welcher

$R^1$ für einen geradkettigen verzweigten oder cyclischen, aliphatischen Kohlenwasserstoffrest mit bis zu 10 C-Atomen steht, der gegebenenfalls substituiert ist durch $C_1$-$C_6$-Alkoxy, $C_1$-$C_6$-Alkylsulfonyl, ein oder mehrere Fluor, Chlor, Brom, Cyano, Hydroxy, durch eine Gruppe der Formel

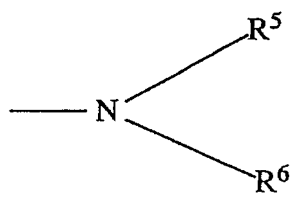

worin

$R^5$ und $R^6$ gleich oder verschieden sind, und

für Wasserstoff,

für $C_1$-$C_6$-Alkyl,

für Phenyl oder Benzyl,

für Acetyl oder Benzoyl stehen,

oder durch Phenyl, Pyridyl, Thienyl, Furyl, Pyrimidyl, Chinolyl oder Isochinolyl, wobei der Phenyl- bzw. die Heteroarylreste bis zu drei gleiche oder verschiedene Substituenten aus der Reihe Fluor, Chlor, Brom, $C_1$-$C_4$-Alkyl, $C_1$-$C_4$-Alkoxy, Cyano, Nitro, Di-$C_1$-$C_4$-alkylamino, oder Trifluormethyl tragen können,

$R^2$ für Wasserstoff, $C_1$-$C_5$-Alkyl, -CN,

$R^3$ für Wasserstoff oder

für geradkettiges oder verzweigtes $C_1$-$C_4$-Alkyl steht,

X für Wasserstoff oder

für Fluor, Chlor oder Brom steht,

Y für die Gruppe

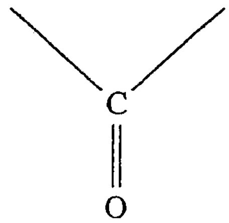

oder $-SO_2$ steht,
und
$R^4$ für einen geradkettigen, verzweigten oder cyclischen, gesättigten oder ungesättigten Kohlenwasserstoffrest mit bis zu 15 Kohlenstoffatomen steht, der gegebenenfalls durch ein oder mehrere Fluor oder Chlor substituiert ist, oder
für Phenyl steht, das gegebenenfalls ein- bis dreifach gleich oder verschieden substituiert durch Fluor, Chlor, Brom, $C_1$-$C_6$-Alkyl, $C_1$-$C_6$-Alkoxy, $C_1$-$C_4$-Halogenalkyl mit bis zu 5 Halogenatomen, Nitro, Cyano, $C_1$-$C_4$-Alkylsulfonyl oder durch eine Gruppe der Formel

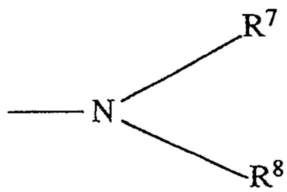

worin
$R^7$, $R^8$ die für $R^5$, $R^6$ angegebene Bedeutung hat und mit diesen gleich oder verschieden sind,
oder
für Benzyl oder Phenethyl steht, oder
für gegebenenfalls durch Fluor, Chlor, $C_1$-$C_4$-Alkyl, $C_1$-$C_4$-Alkoxy, Cyano, Nitro oder Dimethylamino substituiertes Pyridyl, Thienyl, Furyl, Chinolyl oder
Pyrimidyl steht,
oder
für die Gruppe

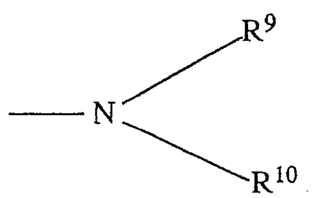

wobei
$R^9$, $R^{10}$ die für $R^5$, $R^6$ angegebene Bedeutung hat und mit diesen gleich oder verschieden sein können,
oder die Gruppe $-Y-R^4$ Wasserstoff bedeutet,
in Form ihrer Isomeren, Isomerengemische, Racemate, optischen Antipoden sowie in Form ihrer physiologisch unbedenklichen Salze.

Die erfindungsgemäßen Stoffe besitzen eine gute kontraktionskraft verstärkende und positiv inotrope Wirkung am Herzen.

Bevorzugt sind Verbindungen der allgemeinen Formel (I), in welcher

$R^1$ für einen geradkettigen, verzweigten oder cyclischen, aliphatischen Kohlenwasserstoffrest mit bis zu 6 C-Atomen steht, der gegebenenfalls substituiert ist durch $C_1$-$C_3$-Alkoxy, Fluor, Chlor, Cyano, Hydroxy, durch eine Gruppe der Formel

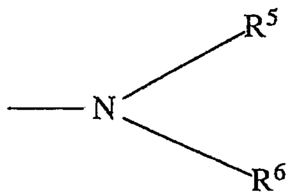

worin
$R^5$ und $R^6$ gleich oder verschieden sind, und
für Wasserstoff,
für $C_1$-$C_4$-Alkyl,
für Phenyl oder Benzyl,
für Acetyl stehen,
oder durch Phenyl, Pyridyl, Pyrimidyl oder Chinolyl, wobei der Phenyl-bzw.
die Heteroarylreste durch Fluor, Chlor, Methyl, Methoxy, Cyano, Nitro oder Trifluormethyl substituiert sein können, $R^2$ für Wasserstoff, $C_1$-$C_4$-Alkyl, -CN,
$R^3$ für Wasserstoff steht,
X für Wasserstoff oder für Fluor steht,
Y für die Gruppe

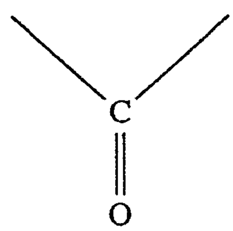

oder -$SO_2$ steht,
und
$R^4$ für einen geradkettigen, verzweigten oder cyclischen, gesättigten oder ungesättigten Kohlenwasserstoffrest mit bis zu 10 Kohlenstoffatomen steht, der gegebenenfalls durch ein oder mehrere Fluor oder Chlor substituiert ist, oder
für Phenyl steht, das gegebenenfalls bis zu zweifach gleich oder verschieden substituiert ist durch Fluor, Chlor, $C_1$-$C_4$-Alkyl, $C_1$-$C_4$-Alkoxy, $C_1$-$C_4$-Halogenalkyl, Nitro, Cyano oder durch Di-$C_1$-$C_4$-alkylamino oder
für Benzyl steht, oder
für gegebenenfalls durch Fluor, Chlor Methyl, Methoxy oder Nitro substituiertes Pyridyl, Furyl, Thienyl oder Chinolyl steht, oder
für die Gruppe der Formel

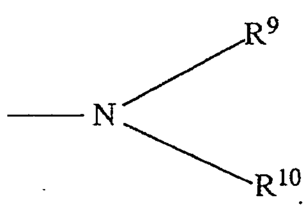

wobei
$R^9$, $R^{10}$ die für $R^5$, $R^6$ angegebene Bedeutung hat und mit diesen gleich oder verschieden sind oder die Gruppe -Y-$R^4$ Wasserstoff bedeutet,
in Form ihrer Isomeren, Isomerengemische, Racemate, optischen Antipoden sowie in Form ihrer physiologisch unbedenklichen Salze.

Die erfindungsgemäßen Stoffe können in Form ihrer Salze vorliegen. Im allgemeinen sind dies Salze der erfindungsgemäßen Stoffe mit anorganischen oder organischen Säuren. Bevorzugt sind jedoch die physiologisch unbedenklichen Salze der erfindungsgemäßen Stoffe mit anorganischen und organischen Säuren. Als Beispiele seien genannt: Hydrohalogenide, Hydrogensulfate, Sulfate, Hydrogenphosphate, Acetate, Maleate, Citrate, Fumarate, Tartrate, Lactate oder Benzoate.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Racemformen lassen sich ebenso wie die Diastereomerengemische in bekannter Weise in die stereoisomer einheitlichen Bestandteile trennen (vgl. E.L. Eliel, Stereochemistry of Carbon Compounds, Mc Graw Hill, 1962).

Die erfindungsgemäßen Verbindungen der allgemeinen Formel (I) erhält man,
wenn man Nitroverbindungen der allgemeinen Formel (II)

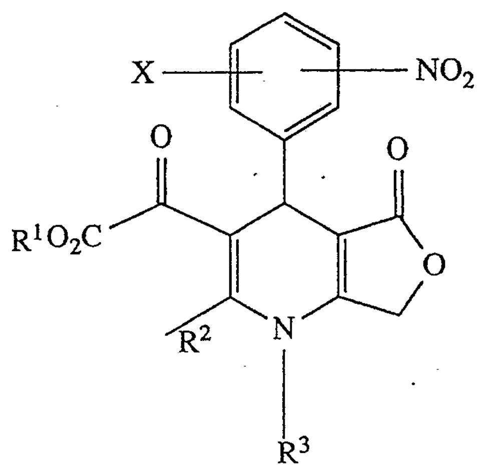

(II)

in welcher
$R^1$, $R^2$, $R^3$ und X die angegebene Bedeutung haben,
zu Aminoverbindungen der allgemeinen Formel (III)

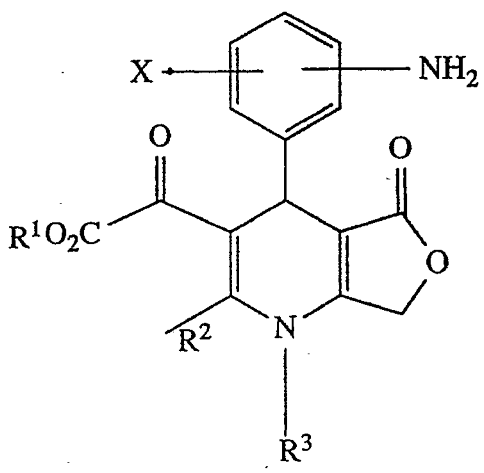

(III)

in welcher
$R^1$, $R^2$, $R^3$ und X die angegebene Bedeutung haben,
durch Hydrierung mit Metallkatalysatoren in Anwesenheit von Säuren bei Temperaturen zwischen -20° C und +100° C in Gegenwart von Wasser oder organischen Lösungsmitteln hydriert und gegebenenfalls in einem zweiten Schritt die Verbindungen der Formel III mit Verbindungen der allgemeinen Formel IV

$$R^4 — Y — Z \qquad (IV)$$

in welcher
$R^4$, Y die angegebene Bedeutung haben und
Y für Halogen,
oder
für eine Gruppe -O-Y-$R^4$ steht,
in Gegenwart von inerten organischen Lösungsmitteln und organischen oder anorganischen Basen bei Temperaturen zwischen 0 und 100° C umsetzt.

Verwendet man als Ausgangsstoffe 2-Methyl-4-(3-nitrophenyl)-5-oxo-1.4.5.7-tetrahydrofuro[3 ,4-b]-pyridin-3-carbonsäure-methylester und Benzoylchlorid bzw. p-Tosylchlorid, läßt sich die Reaktion durch folgendes Schema verdeutlichen:
$R^1$ - für einen geradkettigen, verzweigten oder cyclischen, aliphatischen Kohlenwasserstoffrest mit bis zu 6 Kohlenstoffatomen steht, der gegebenenfalls substituiert ist durch $C_1$-$C_3$-Alkoxy, Fluor, Chlor, Cyano, Hydroxy, die Gruppe der Formel

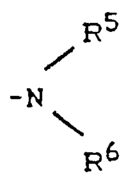

worin
$R^5$, $R^6$

- gleich oder verschieden sind und
- für Wasserstoff,
- für $C_1$-$C_4$-Alkyl,
- für Phenyl oder Benzyl, oder
- für Acetyl stehen,

oder durch Phenyl, Pyridyl, Chinolyl oder Pyrimidyl, wobei der Phenyl- bzw. die Heteroarylreste durch Fluor, Chlor, Methyl, Methoxy, Cyano, Nitro oder Trifluormethyl substituiert sein können,
$R^2$ - für Wasserstoff, $C_1$-$C_4$-Alkyl, Cyano,
$R^3$ - für Wasserstoff steht,
X - für Wasserstoff oder Fluor steht,
Y - für die Gruppe

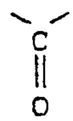

oder $-SO_2-$ steht,
und
R4 - für einen geradkettigen, verzweigten oder cyclischen, Alkyl- oder Alkenylrest mit bis zu 10 Kohlenstoffatomen steht, der gegebenenfalls durch ein oder mehrere Fluor oder Chlor substituiert ist, oder
- für Phenyl steht, das gegebenenfalls bis zu zweifach gleich oder verschieden substituiert ist durch Fluor, Chlor, $C_1$-$C_4$-Alkyl, $C_1$-$C_4$-Alkoxy, $C_1$-$C_4$-Halogenalkyl, Nitro, Cyano oder durch Di-$C_1$-$C_4$-alkylamino, oder
- für Benzyl steht, oder
- für gegegebenenfalls durch Fluor, Chlor, Methyl, Methoxy oder Nitro substituiertes Pyridyl, Furyl, Thienyl oder Chinolyl steht oder
- für die Gruppe der Formel

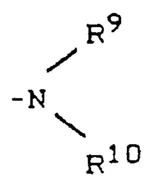

worin
$R^9$ , $R^{10}$

- die für $R^5$ , $R^6$ angegebene Bedeutung haben und mit diesen gleich oder verschieden sind

oder die Gruppe $-Y-R^4$ Wasserstoff bedeutet,
in Form ihrer Isomeren, Isomerengemische, Racemate, optischen Antipoden sowie in Form ihrer physiologisch unbedenklichen Salze.

Die erfindungsgemäßen Stoffe können in Form ihrer Salze vorliegen. Im allgemeinen sind dies Salze der erfindungsgemäßen Stoffe mit anorganischen oder organischen Säuren. Bevorzugt sind jedoch die physiologisch unbedenklichen Salze der erfindungsgemäßen Stoffe mit anorganischen und organischen Säuren. Als Beispiele seien genannt: Hydrohalogenide, Hydrogensulfate, Sulfate, Hydrogenphosphate, Acetate, Maleate, Citrate, Fumarate, Tartrate, Lactate oder Benzoate.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Racemformen lassen sich ebenso wie die Diastereomerengemische in bekannter Weise in die stereoisomer einheitlichen Bestandteile trennen (vgl. E.L. Eliel, Stereochemistry of Carbon Compounds, Mc Graw Hill, 1962).

Die erfindungsgemäßen Verbindungen der allgemeinen Formel (I), erhält man,

wenn man Nitroverbindungen der allgemeinen Formel (II),

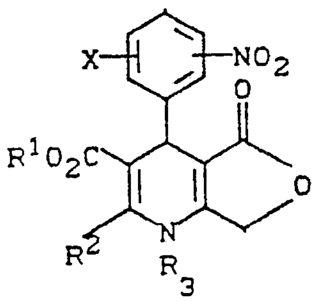

(II)

in welcher
$R^1$, $R^2$, $R^3$ und X die angegebene Bedeutung haben,
gegebenenfalls in Anwesenheit eines Katalysators, gegebenenfalls in Gegenwart einer Säure und gegebenenfalls in Gegenwart eines inerten Lösungsmittels in an sich bekannter Weise zu Aminoverbindungen der allgemeinen Formel (III)

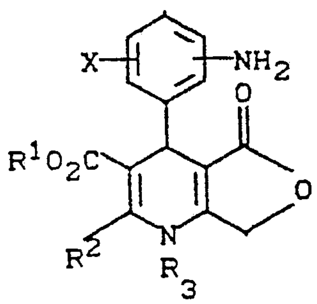

(III)

in welcher
$R^1$, $R^2$, $R^3$ und X die angegebene Bedeutung haben
reduziert und gegebenenfalls in einem zweiten Schritt die Verbindungen der Formel (III) mit Verbindungen der allgemeinen Formel IV

$$R^4 - Y - Z \qquad (IV)$$

in welcher
$R^4$, Y die angegebene Bedeutung haben und
Z für Halogen, bevorzugt Chlor oder Brom, oder für eine Gruppe - O-Y-$R^4$ steht,
gegebenenfalls in Anwesenheit einer Base, gegebenenfalls in Gegenwart eines inerten Lösungsmittels umsetzt.

Verwendet man als Ausgangsstoffe 2-Methyl-4-(3-nitrophenyl)-5-oxo-1.4.5.7-tetrahydrofuro[3,4-b]pyridin-3-carbonsäure-methylester und Benzoylchlorid bzw. p-Tosylchlorid, läßt sich die Reaktion durch folgendes Schema verdeutlichen:

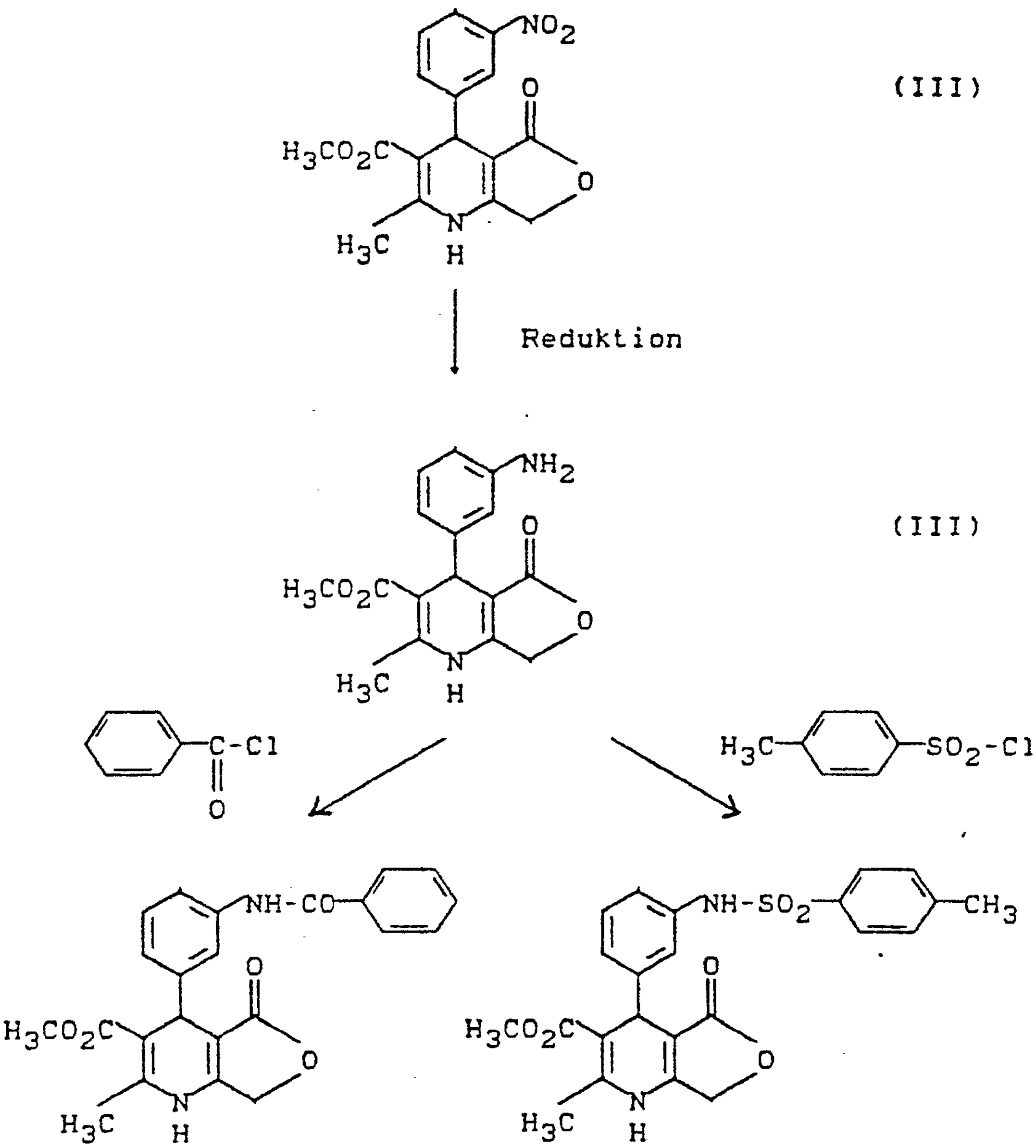


(III)

(III)

Die als Ausgangsstoffe verwendeten Verbindungen der Formel (II) sind bekannt oder können nach bekannten Methoden hergestellt werden (DE-OS 32 06 671).

Die Reduktion im ersten Reaktionsschritt erfolgt in an sich bekannter Weise, bevorzugt durch Hydrierung mit Metallkatalysatoren wie beispielsweise Platin, Palladium, Palladium auf Tierkohle oder Raney-Nickel in Anwesenheit von Säuren.

Als Säuren können erfindungsgemäß starke Mineralsäuren aber auch organische Säuren eingesetzt werden. Bevorzugt sind Halogenwasserstoffsäuren wie HCl oder HBr, Schwefelsäure, phosphorsäure, Perchlorsäure, Essigsäure, Trifluoressigsäure oder p-Toluolsulfonsäure.

Der Katalysator wird hierbei im allgemeinen in einer Menge von 0,1 bis 50 Mol %, bevorzugt von 1 bis 10 Mol %, bezogen auf die Nitroverbindung eingesetzt.

Die Hydrierung erfolgt im allgemeinen im Temperaturbereich von -20 bis +100 °C, bevorzugt im Bereich von 0 bis +50 °C.

Im allgemeinen erfolgt die Hydrierung mit einem Überdruck von 5 bis 100 bar, bevorzugt von 10 bis 80 bar. Es ist ebenso möglich, die Hydrierung bei Normaldruck durchzuführen.

Als Lösungsmittel für die Hydrierung eignen sich Wasser und/oder inerte organische Lösungsmittel. Bevorzugt gehören hierzu Alkohole wie Methanol, Ethanol, Propanol oder Isopropanol, Ether wie Diethylether, Dioxan, Tetrahydrofuran, Glykolmonomethylether oder Glykoldimethylether, Chlorkohlenwasserstoffe wie Methylenchlorid, Chloroform oder Tetrachlorkohlenstoff, Eisessig, Dimethylformamid, Essigester oder Aceton. Ebenso ist es möglich Gemische der genannten Lösungsmittel einzusetzen.

Besonders bevorzugt wird die Reduktion mit Raney-Nickel in Alkoholen mit Wasserstoffüberdruck durchgeführt.

Die Reduktion kann aber ebenso mit Metallen wie Zink, Zinn oder Eisen in Beisein von Säuren wie Essigsäure oder Salzsäure wie es von R. Schröter in Houben-Weyls "Methoden der organischen Chemie" XI/1, S 363 ff beschrieben wird, durchgeführt werden.

Als Lösungsmittel im zweiten Reaktionsschritt eignen sich inerte organische Lösungsmittel, die sich unter den Reaktionsbedingungen nicht verändern. Hierzu gehören bevorzugt Ether wie Diethylether, Dioxan, Tetrahydrofuran, Glykolmonomethylether oder Glykoldimethylether, Chlorkohlenwasserstoffe wie Methylenchlorid, Chloroform, Tetrachlorkohlenstoff oder 1,2-Dichlorethan, oder Kohlenwasserstoffe wie Benzol, Toluol, Xylol, Hexan oder Erdölfraktionen. Ebenso ist es möglich, Gemische der genannten Lösungsmittel einzusetzen.

Als Basen eignen sich die üblichen organischen Basen. Hierzu gehören bevorzugt Trialkylamine wie Triethylamin oder Pyridin, Chinolin, Isochinolin, Methylpiperidin oder Methylmorpholin. Ebenso ist es möglich, anorganische Basen wie Natrium- oder Kaliumcarbonat, oder aber Alkoholate wie Natriumethylat, Natriummethylat, Kaliumethylat oder Kaliummethylat zu verwenden. Besonders bevorzugt wird Triethylamin eingesetzt.

Die Reaktion wird in einem Temperaturbereich von 0° C bis 100° C, bevorzugt von 10 bis 50° C, durchgeführt.

Die Umsetzung kann bei Normaldruck, aber auch bei erhöhtem oder erniedrigtem Druck durchgeführt werden. Im allgemeinen arbeitet man bei Normaldruck.

Die erfindungsgemäßen Verbindungen zeigen ein nicht vorhersehbares und wertvolles pharmakologisches Wirkungsspektrum. Sie können als Cardiotonika zur Verbesserung der Herzkontraktilität dienen. Darüber hinaus können sie als Antihypotonika, zur Senkung des Blutzuckers, zur Abschwellung von Schleimhäuten und zur Beeinflussung des Salz- und Flüssigkeitshaushaltes eingesetzt werden.

Die Herzkontraktilität verstärkende Wirkung wurde an isolierten Vorhöfen von Meerschweinchenherzen gefunden.

Dazu werden die linken Vorhöfe von Meerschweinchenherzen isoliert und in ein thermostatisiertes Organbad gehängt, welches eine isotonische Mineralsalzlösung, die dem Ionenmilieu und dem pH-Wert von Körperflüssigkeiten angepaßt ist, mit geeigneten Nährstoffen enthält. Dieses Organbad wurde mit einem Gasgemisch bestehend aus Sauerstoff und Kohlendioxid begast, wobei der Kohlendioxidgehalt so bemessen ist, daß der pH-Wert des Organbades konstant bleibt, Die linken Vorhöfe wurden in das Organbad eingespannt, die Spannung mittels eines Kraftaufnehmers registriert, wobei ein bestimmter Grundtonus eingestellt wird, Anschließend wurden die linken Vorhofe kontinuierlich in bestimmten Abständen elektrisch gereizt und die dabei erfolgenden Kontraktionen registriert. Bei einer Konzentration von $10^{-6}$ g/ml kommt es zu einer Steigerung der Kontraktionskraft gegenüber dem gleich 100 % gesetzten Ausgangswert.

| Beispiel-Nr. | Kontrakt.-Steigerung bei $10^{-6}$ g/ml |
|---|---|
| 6 | + 47 % |
| 7 | + 80 % |
| 8 | + 42 % |
| 28 | + 33 % |
| 29 | + 140 % |

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstofffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin, Sulfitablaugen, Methylencellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendung gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Herstellungsbeispiele

Beispiel 1

4-(2-Aminophenyl)-2-methyl-5-oxo-1.4.5.7-tetrahydrofuro[3.4-b]pyridin-3-carbonsäureethylester.

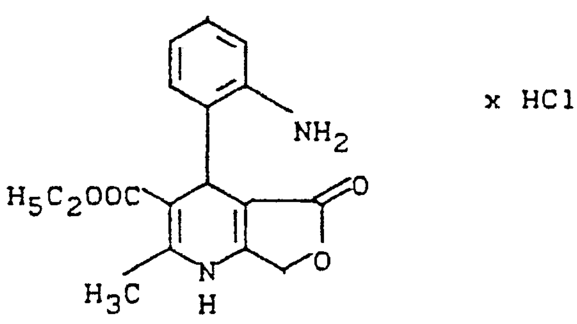

58 mmol 4-(2-Nitrophenyl)-2-methyl-5-oxo-1.4.5.7-tetrahydrofuro[3.4-b]pyridin-3-carbonsäureethylester werden in 200 ml Tetrahydrofuran gelöst und mit 2 g Raney-Nickel versetzt. Bei 50 bar $H_2$-Druck wird 1,5 h hydriert. Die Lösung wird eingedampft, mit verdünnter Salzsäure versetzt, abgesaugt und getrocknet.

Ausbeute: 56 % der Theorie
Schmelzpunkt: 175-183 °C

Analog Beispiel 1 wurden hergestellt:

Beispiel 2

4-(2-Aminophenyl)-2-methyl-5-oxo-1.4-5.7-tetrahydrofuro-[3.4-b]pyridin-3-carbonsäuremethylester

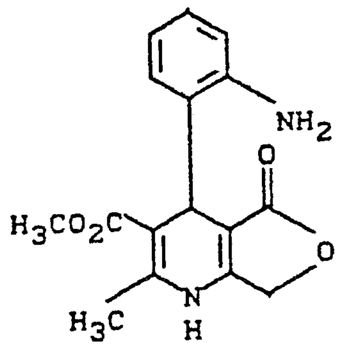

Ausbeute: 80 % der Theorie
Schmelzpunkt: 193-5 °C

Beispiel 3

4-(2-Aminophenyl)-2-methyl-5-oxo-1.4.5.7-tetrahydrofuro-[3.4-b]pyridin-3-carbonsäure-butylester

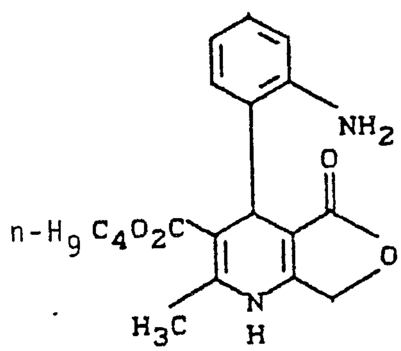

Ausbeute: 60 % der Theorie
Schmelzpunkt: 167-9 °C

Beispiel 4

4-(3-Aminophenyl)-2-methyl-5-oxo-1.4.5.7-tetrahydrofuro-[3.4-b]pyridin-3-carbonsäureethylester

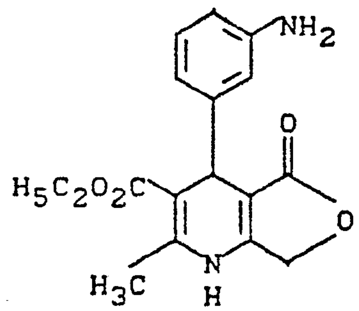

Ausbeute: 80 % der Theorie
Schmelzpunkt: 179-181 °C

Beispiel 5

4-(2-Benzoylamino)-2-methyl-5-oxo-1.4.5.7-tetrahydrofuro-[3.4-b]pyridin-3-carbonsäureethylester

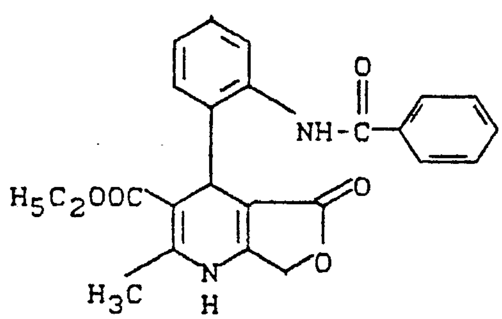

10 mmol 4-(2-Aminophenyl)-2-methyl-5-oxo-12.4.5.7-tetrahydrofuro[3.4-b]pyridin-3-carbonsäureethylester werden in 100 ml $CH_2Cl_2$ gelöst und mit 3,3 ml Triethylamin (20 mmol) versetzt. Zu dieser Lösung gibt man 10 mmol Benzoylchlorid. Es wird 1.5 - 2.0 h bei Raumtemperatur gerührt. Die Lösung wird 1 x mit $H_2O$ ausgeschüttelt, die organische Phase mit $Na_2SO_4$ oder Mol-Sieb getrocknet und eingeengt. Aus Methanol oder Ethanol erhält man Kristalle.

Ausbeute: 80 % der Theorie
Schmelzpunkt: 275 °C

Analog Beispiel 5 wurden die Beispiele in den folgenden Tabellen hergestellt:

Tabelle 1

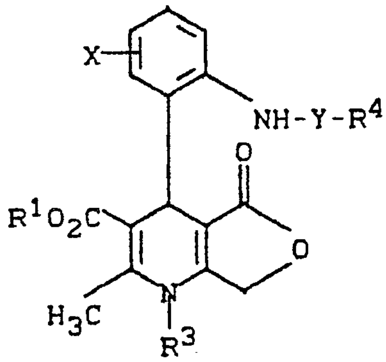

| Bsp.-. Nr. | $R^1$ | $R^3$ | X | Y | $R^4$ | Schmelz-punkt [°C] |
|---|---|---|---|---|---|---|
| 6 | $CH_3$ | H | H | CO | 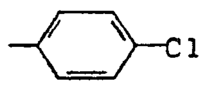 | > 270 |
| 7 | $CH_3$ | H | H | CO | 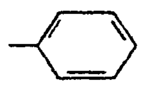 | > 270 |
| 8 | $CH_3$ | H | H | CO | 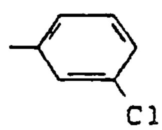 | > 270 |
| 9 | $CH_3$ | H | H | CO | 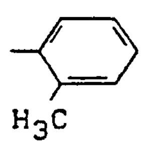 | 235-6 |
| 10 | $CH_3$ | H | H | CO | 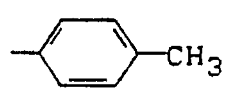 | 220-2 |

Tabelle 1 (Fortsetzung)

| Bsp.-. Nr. | $R^1$ | $R^3$ | X | Y | $R^4$ | Schmelzpunkt [°C] |
|---|---|---|---|---|---|---|
| 11 | $CH_3$ | H | H | CO | 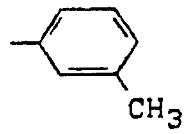 | › 270 |
| 12 | $CH_2CH_3$ | H | H | $SO_2$ | 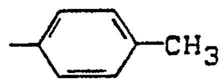 | 115-20 |
| 13 | $CH_2CH_3$ | H | H | CO | 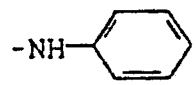 | 233-7 |
| 14 | $CH_2CH_3$ | H | H | CO | 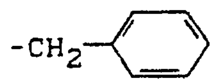 | › 250 |
| 15 | $CH_2CH_3$ | H | H | CO | 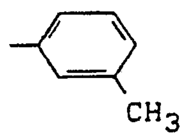 | › 250 |
| 16 | $CH_2CH_3$ | H | H | CO | 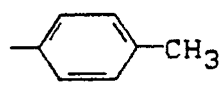 | › 250 |

Tabelle 1 (Fortsetzung)

| Bsp.-. Nr. | $R^1$ | $R^3$ | X | Y | $R^4$ | Schmelz- punkt [°C] |
|---|---|---|---|---|---|---|
| 17 | $CH_2CH_3$ | H | H | CO | 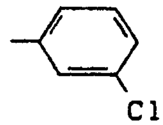 | > 250 |
| 18 | $CH_2CH_3$ | H | H | CO | 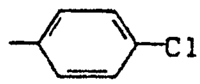 | > 250 |
| 19 | $CH_2CH_3$ | H | H | CO | $CH_2CH_3$ | > 250 |
| 20 | $CH_2CH_3$ | H | H | CO | $-CH_2-CH(CH_3)_2$ | 228-30 |
| 21 | $CH_2CH_3$ | H | H | CO | 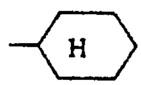 | 183-5 |
| 22 | $CH_2CH_3$ | H | H | CO | $-(CH_2)_7CH_3$ | 167-70 |

Tabelle 1 (Fortsetzung)

| Bsp.-. Nr. | $R^1$ | $R^3$ | X | Y | $R^4$ | Schmelzpunkt [°C] |
|---|---|---|---|---|---|---|
| 23 | $CH_2CH_3$ | H | H | CO | 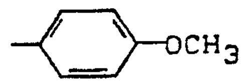 | > 250 |
| 24 | $CH_2CH_3$ | H | H | CO | 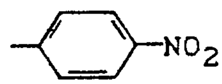 | > 250 |
| 25 | $CH_2CH_3$ | H | H | CO | 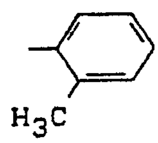 | > 250 |
| 26 | $CH_2CH_3$ | H | H | CO | 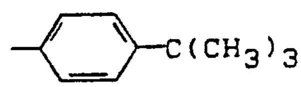 | > 250 |
| 27 | $CH_2CH_3$ | H | H | CO | 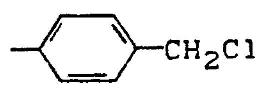 | > 250 |

Tabelle 1 (Fortsetzung)

| Bsp.-. Nr. | $R^1$ | $R^3$ | X | Y | $R^4$ | Schmelz-punkt [°C] |
|---|---|---|---|---|---|---|
| 28 | $CH_2CH_3$ | H | H | CO | 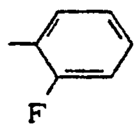 | > 250 |
| 29 | $CH_2CH_3$ | H | H | CO | 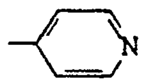 | > 250 |
| 30 | $CH_2CH_3$ | H | H | CO | 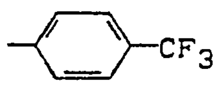 | > 250 |
| 31 | $n-C_4H_9$ | H | H | CO | 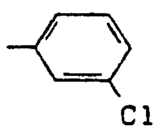 | 232-5 |
| 32 | $n-C_4H_9$ | H | H | CO | 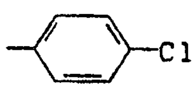 | 225-7 |

Tabelle 1 (Fortsetzung)

| Bsp.-. Nr. | $R^1$ | $R^3$ | X | Y | $R^4$ | Schmelz-punkt [°C] |
|---|---|---|---|---|---|---|
| 33 | n-$C_4H_9$ | H | H | CO | 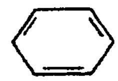 | 148-55 |
| 34 | n-$C_4H_9$ | H | H | CO | 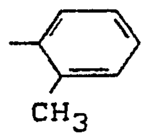 | 225-27 |
| 35 | n-$C_4H_9$ | H | H | CO | 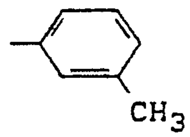 | 203-4 |
| 36 | n-$C_4H_9$ | H | H | CO | 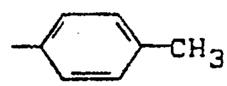 | 234-6 |

Tabelle 2

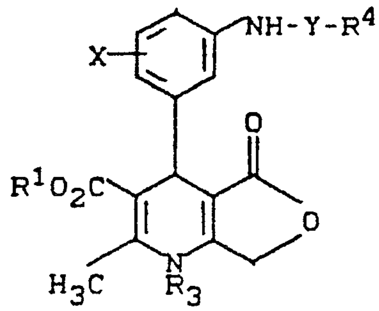

| Bsp.-Nr. | $R^1$ | $R^3$ | X | Y | $R^4$ | Schmelzpunkt [°C] |
|---|---|---|---|---|---|---|
| 37 | $C_2H_5$ | H | H | CO | 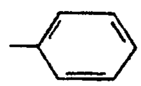 | 252-3 |
| 38 | $C_2H_5$ | H | H | CO | 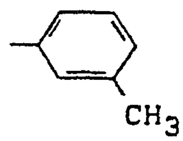 | 225-8 |
| 39 | $C_2H_5$ | H | H | CO | 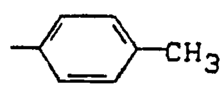 | 251-6 |

**Ansprüche**

1. 4-Aminoaryldihydropyridine der allgemeinen Formel (I)

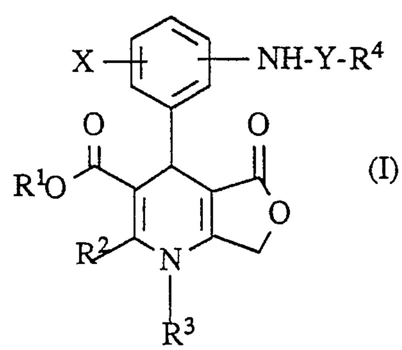

(I)

in welcher
$R^1$ für einen geradkettigen, verzweigten oder cyclischen, aliphatischen Kohlenwasserstoffrest mit bis zu 10 C-Atomen steht, der gegebenenfalls substituiert ist durch $C_1$-$C_6$-Alkoxy, $C_1$-$C_6$-Alkylsulfonyl, ein oder mehrere Fluor, Chlor, Brom, Cyano, Hydroxy, durch eine Gruppe der Formel

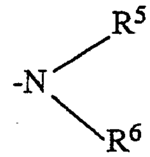

worin

$R^5$ und $R^6$ gleich oder verschieden sind, und

für Wasserstoff,

für $C_1$-$C_6$-Alkyl,

für Phenyl oder Benzyl,

für Acetyl oder Benzoyl stehen,

oder durch Phenyl, Pyridyl, Thienyl, Furyl, Pyrimidyl, Chinolyl oder Isochinolyl, wobei der Phenyl- bzw. die Heteroarylreste bis zu drei gleiche oder verschiedene Substituenten aus der Reihe Fluor, Chlor, Brom, $C_1$-$C_4$-Alkyl, $C_1$-$C_4$-Alkoxy, Cyano, Nitro, Di-$C_1$-$C_4$-alkylamino, oder Trifluormethyl tragen können,

$R^2$ für Wasserstoff, $C_1$-$C_5$-Alkyl, -CN,

$R^3$ für Wasserstoff oder

für geradkettiges oder verzweigtes $C_1$-$C_4$-Alkyl steht,

X für Wasserstoff oder

für Fluor, Chlor oder Brom steht,

Y für die Gruppe

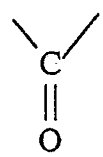

oder $-SO_2$ steht,

und

$R^4$ für einen geradkettigen, verzweigten oder cyclischen, gesättigten oder ungesättigten Kohlenwasserstoffrest mit bis zu 15 Kohlenstoffatomen steht, der gegebenenfalls durch ein oder mehrere Fluor oder Chlor substituiert ist, oder

für Phenyl steht, das gegebenenfalls ein- bis dreifach gleich oder verschieden substituiert ist durch Fluor, Chlor, Brom, $C_1$-$C_6$-Alkyl, $C_1$-$C_6$-Alkoxy, $C_1$-$C_4$-Halogenalkyl mit bis zu 5 Halogenatomen, Nitro, Cyano, $C_1$-$C_4$-Alkylsulfonyl oder durch eine Gruppe der Formel

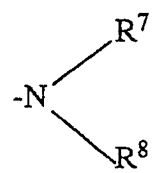

worin

$R^7$, $R^8$ die für $R^5$, $R^6$ angegebene Bedeutung hat und mit diesen gleich oder verschieden sind,

oder

für Benzyl oder Phenethyl steht, oder

für gegebenenfalls durch Fluor, Chlor, $C_1$-$C_4$-Alkyl, $C_1$-$C_4$-Alkoxy, Cyano, Nitro oder Dimethylamino substituiertes Pyridyl, Thienyl, Furyl, Chinolyl oder Pyrimidyl steht,

oder

für die Gruppe

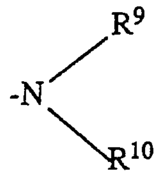

wobei
$R^9$, $R^{10}$ die für $R^5$, $R^6$ angegebene Bedeutung hat und mit diesen gleich oder verschieden sein können,.
oder die Gruppe -Y-$R^4$ Wasserstoff bedeutet,
in Form ihrer Isomeren, Isomerengemische, Racemate, optischen Antipoden sowie in Form ihrer physiologisch unbedenklichen Salze.

**2.** 4-Aminoaryldihydropyridine der allgemeinen Formel (I) in Anspruch 1, in welcher
$R^1$ für einen geradkettigen, verzweigten oder cyclischen, aliphatischen Kohlenwasserstoffrest mit bis zu 6 C-Atomen steht, der gegebenenfalls substituiert ist durch $C_1$-$C_3$-Alkoxy, Fluor, Chlor, Cyano, Hydroxy, durch eine Gruppe der Formel

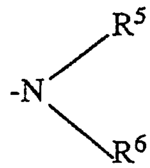

worin
$R^5$, $R^6$ gleich oder verschieden sind, und
für Wasserstoff,
für $C_1$-$C_4$-Alkyl,
für Phenyl oder Benzyl,
für Acetyl stehen,
oder durch Phenyl, Pyridyl, Pyrimidyl oder Chinolyl, wobei der Phenyl-bzw die Heteroarylreste durch Fluor, Chlor, Methyl, Methoxy, Cyano, Nitro oder Trifluormethyl substituiert sein können,
$R^2$ für Wasserstoff, $C_1$-$C_4$-Alkyl, -CN,
$R^3$ für Wasserstoff steht,
X für Wasserstoff oder für Fluor steht,
Y für die Gruppe

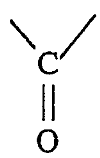

oder -$SO_2$ steht,
und
$R^4$ für einen geradkettigen, verzweigten oder cyclischen, gesättigten oder ungesättigten Kohlenwasserstoffrest mit bis zu 10 Kohlenstoffatomen steht, der gegebenenfalls durch ein oder mehrere Fluor oder Chlor substituiert ist, oder
für Phenyl steht, das gegebenenfalls bis zu zweifach gleich oder verschieden substituiert ist durch Fluor, Chlor, $C_1$-$C_4$-Alkyl, $C_1$-$C_4$-Alkoxy, $C_1$-$C_4$-Halogenalkyl, Nitro, Cyano oder durch Di-$C_1$-$C_4$-alkylamino oder
für Benzyl steht, oder

für gegebenenfalls durch Fluor, Chlor, Methyl, Methoxy oder Nitro substituiertes Pyridyl, Furyl, Thienyl oder Chinolyl steht, oder
für die Gruppe der Formel

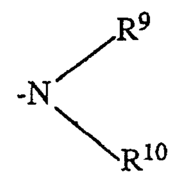

wobei
$R^9$, $R^{10}$ die für $R^5$, $R^6$ angegebene Bedeutung hat und mit diesen gleich oder verschieden sind oder die Gruppe -Y-$R^4$ Wasserstoff bedeutet,
in Form ihrer Isomeren, Isomerengemische, Racemate, optischen Antipoden sowie in Form ihrer physiologisch unbedenklichen Salze.

**3.** 4-Aminoaryldihydropyridin gemäß Ansprüche 1-2 zur Bekämpfung von Erkrankungen.

**4.** Arzneimittel enthaltend als Wirkstoff 4-Aminoaryldihydropyridine gemäß Ansprüche 1-2.

**5.** Verfahren zur Herstellung von 4-Aminoaryl-dihydropyridine der allgemeinen Formel (I)

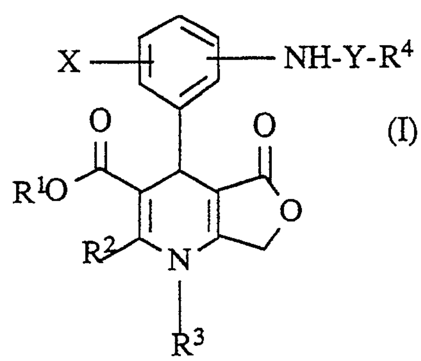

(I)

in welcher $R^1$,$R^2$,$R^3$ $R^4$ X und Y die in Anspruch 1 angegebenen Bedeutungen haben, dadurch gekennzeichnet, daß man Nitroverbindungen der Formel II

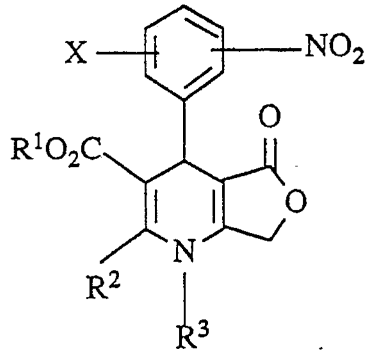

(II)

in welcher

$R^1$, $R^2$, $R^3$ und X die angegebene Bedeutung haben, zu Aminoverbindungen der allgemeinen Formel (III)

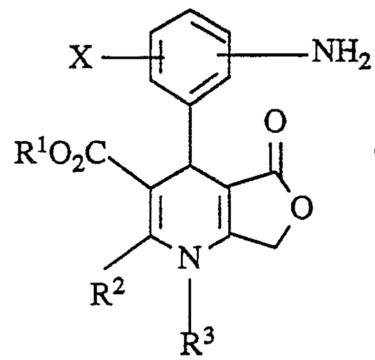

(III)

in welcher
$R^1$, $R^2$, $R^3$ und X die angegebene Bedeutung haben,
durch Hydrierung mit Metallkatalysatoren in Anwesenheit von Säuren bei Temperaturen zwischen -20 °C und +100 °C in Gegenwart von Wasser oder organischen Lösungsmitteln hydriert und gegebenenfalls in einem zweiten Schritt die Verbindungen der Formel III mit Verbindungen der allgemeinen Formel IV

$$R^4 - Y - Z \qquad (IV)$$

in welcher
$R^4$, Y die angegebene Bedeutung haben und
Z für Halogen,
oder
für eine Gruppe -O-Y-$R^4$ steht,
in Gegenwart von inerten organischen Lösungsmitteln und organischen oder anorganischen Basen bei Temperaturen zwischen 0 und 100 °C umsetzt.

6. Verfahren gemäß Anspruch 5, dadurch gekennzeichnet, daß man die Reduktion der Nitroverbindungen der Formel II zu Aminoverbindungen der Formel III in Gegenwart von Raneynickel in Alkoholen mit Wasserstoffüberdruck durchführt.

7. Verfahren gemäß Anspruch 5, dadurch gekennzeichnet, daß man den ersten Reaktionsschritt bei Temperaturen zwischen 0 und 50 °C und den zweiten Reaktionsschritt bei Temperaturen zwischen 10 und 50 °C durchführt.

8. Verfahren zur Herstellung eines Arzneimittels gemäß Anspruch 4, dadurch gekennzeichnet, daß man Verbindungen gemäß Anspruch 1 gegebenenfalls unter Verwendung von Hilfs- und Trägerstoffen in üblicher Weise in eine Applikationsform überführt.

9. Verwendung von Verbindungen der allgemeinen Formel I gemäß Anspruch 1 zur Herstellung von Arzneimitteln mit cardiotonischer Wirkung, zur Blutdruckerhöhung, zur Senkung des Blutzuckers, zur Abschwellung von Schleimhäuten und zur Beeinflussung des Salz- und Flüssigkeitshaushaltes.

Patentanspruch für folgenden Vertragsstaat: ES

1. Verfahren zur Herstellung von 4-Aminoaryl-dihydropyridine der allgemeinen Formel (I)

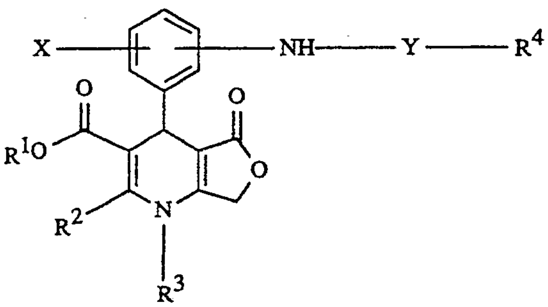

(I)

in welcher

$R^1$ für einen geradkettigen verzweigten oder cyclischen, aliphatischen Kohlenwasserstoffrest mit bis zu 10 C-Atomen steht, der gegebenenfalls substituiert ist durch $C_1$-$C_6$-Alkoxy, $C_1$-$C_6$-Alkylsulfonyl, ein oder mehrere Fluor, Chlor, Brom, Cyano, Hydroxy, durch eine Gruppe der Formel

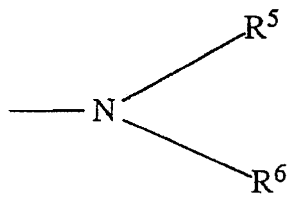

worin

$R^5$ und $R^6$ gleich oder verschieden sind, und

für Wasserstoff,

für $C_1$-$C_6$-Alkyl,

für Phenyl oder Benzyl,

für Acetyl oder Benzoyl stehen,

oder durch Phenyl, Pyridyl, Thienyl, Furyl, Pyrimidyl, Chinolyl oder Isochinolyl, wobei der Phenyl- bzw. die Heteroarylreste bis zu drei gleiche oder verschiedene Substituenten aus der Reihe Fluor, Chlor, Brom, $C_1$-$C_4$-Alkyl, $C_1$-$C_4$-Alkoxy, Cyano, Nitro, Di-$C_1$-$C_4$-alkylamino, oder Trifluormethyl tragen können,

$R^2$ für Wasserstoff, $C_1$-$C_5$-Alkyl, -CN,

$R^3$ für Wasserstoff oder

für geradkettiges oder verzweigtes $C_1$-$C_4$-Alkyl steht,

X für Wasserstoff oder

für Fluor, Chlor oder Brom steht,

Y für die Gruppe

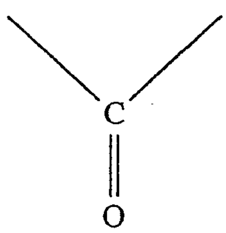

oder $-SO_2$ steht,

und

$R^4$ für einen geradkettigen, verzweigten oder cyclischen, gesättigten oder ungesättigten Kohlenwasserstoffrest mit bis zu 15 Kohlenstoffatomen steht, der gegebenenfalls durch ein oder mehrere Fluor oder Chlor substituiert ist, oder

für Phenyl steht, das gegebenenfalls ein- bis dreifach gleich oder verschieden substituiert durch Fluor,

Chlor, Brom, $C_1$-$C_6$-Alkyl, $C_1$-$C_6$-Alkoxy, $C_1$-$C_4$-Halogenalkyl mit bis zu 5 Halogenatomen, Nitro, Cyano, $C_1$-$C_4$-Alkylsulfonyl oder durch eine Gruppe der Formel

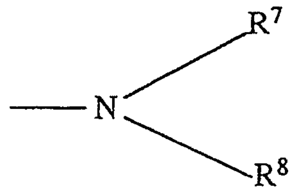

worin
$R^7$, $R^8$ die für $R^5$, $R^6$ angegebene Bedeutung hat und mit diesen gleich oder verschieden sind,
oder
für Benzyl oder Phenethyl steht, oder
für gegebenenfalls durch Fluor, Chlor, $C_1$-$C_4$-Alkyl, $C_1$-$C_4$-Alkoxy, Cyano, Nitro oder Dimethylamino substituiertes Pyridyl, Thienyl, Furyl, Chinolyl oder Pyrimidyl steht,
oder
für die Gruppe

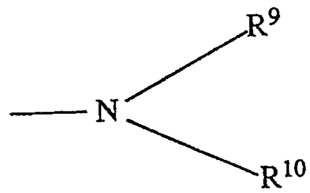

wobei
$R^9$, $R^{10}$ die für $R^5$, $R^6$ angegebene Bedeutung hat und mit diesen gleich oder verschieden sein können,
oder die Gruppe -Y-$R^4$ Wasserstoff bedeutet,
in Form ihrer Isomeren, Isomerengemische, Racemate, optischen Antipoden sowie in Form ihrer physiologisch unbedenklichen Salze,
dadurch gekennzeichnet, daß man Nitroverbindungen der allgemeinen Formel (II)

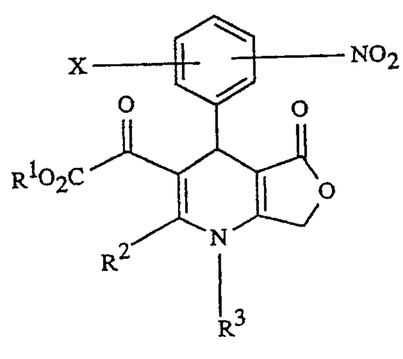

(II)

in welcher
$R^1$, $R^2$, $R^3$ und X die angegebene Bedeutung haben,
zu Aminoverbindungen der allgemeinen Formel (III)

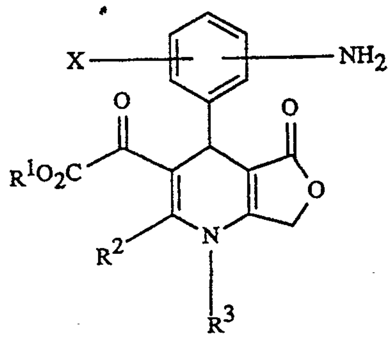

(III)

in welcher
$R^1$, $R^2$, $R^3$ und X die angegebene Bedeutung haben,
durch Hydrierung mit Metallkatalysatoren in Anwesenheit von Säuren bei Temperaturen zwischen -20 °C und +100 °C in Gegenwart von Wasser oder organischen Lösungsmitteln hydriert und gegebenenfalls in einem zweiten Schritt die Verbindungen der Formel III mit Verbindungen der allgemeinen Formel IV

$$R^4 — Y — Z \qquad (IV)$$

in welcher
$R^4$, Y die angegebene Bedeutung haben und
Y für Halogen,
oder
für eine Gruppe -O-Y-$R^4$ steht,
in Gegenwart von inerten organischen Lösungsmitteln und organischen oder anorganischen Basen bei Temperaturen zwischen 0 und 100 °C umsetzt.

**2.** Verfahren gemäß Anspruch 1 zur Herstellung von 4-Aminoaryldihydropyridinen der allgemeinen Formel (I) gemäß Anspruch 1, in welcher
$R^1$ für einen geradkettigen, verzweigten oder cyclischen, aliphatischen Kohlenwasserstoffrest mit bis zu 6 C-Atomen steht, der gegebenenfalls substituiert ist durch $C_1$-$C_3$-Alkoxy, Fluor, Chlor, Cyano, Hydroxy, durch eine Gruppe der Formel

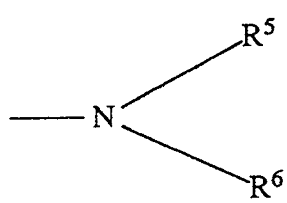

worin
$R^5$ und $R^6$ gleich oder verschieden sind, und
für Wasserstoff,
für $C_1$-$C_4$-Alkyl,
für Phenyl oder Benzyl,
für Acetyl stehen,
oder durch Phenyl, Pyridyl, Pyrimidyl oder Chinolyl, wobei der Phenyl-bzw. die Heteroarylreste durch Fluor, Chlor, Methyl, Methoxy, Cyano, Nitro oder Trifluormethyl substituiert sein können,
$R^2$ für Wasserstoff, $C_1$-$C_4$-Alkyl, -CN,
$R^3$ für Wasserstoff steht,
X für Wasserstoff oder für Fluor steht,
Y für die Gruppe

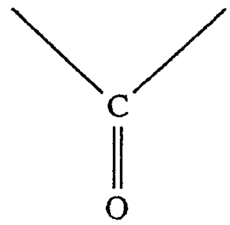

oder $-SO_2$ steht,
und
$R^4$ für einen geradkettigen, verzweigten oder cyclischen, gesättigten oder ungesättigten Kohlenwasserstoffrest mit bis zu 10 Kohlenstoffatomen steht, der gegebenenfalls durch ein oder mehrere Fluor oder Chlor substituiert ist, oder
für Phenyl steht, das gegebenenfalls bis zu zweifach gleich oder verschieden substituiert ist durch Fluor, Chlor, $C_1$-$C_4$-Alkyl, $C_1$-$C_4$-Alkoxy, $C_1$-$C_4$-Halogen alkyl, Nitro, Cyano oder durch Di-$C_1$-$C_4$-alkylamino oder
für Benzyl steht, oder
für gegebenenfalls durch Fluor, Chlor Methyl, Methoxy oder Nitro substituiertes Pyridyl, Furyl, Thienyl oder Chinolyl steht, oder
für die Gruppe der Formel

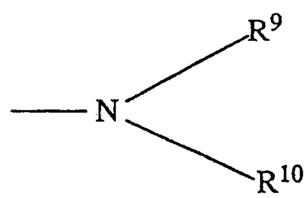

wobei
$R^9$, $R^{10}$ die für $R^5$, $R^6$ angegebene Bedeutung hat und mit diesen gleich oder verschieden sind oder die Gruppe $-Y-R^4$ Wasserstoff bedeutet.

3. Verfahren gemäß Anspruch 1, dadurch gekennzeichnet, daß man die Reduktion der Nitroverbindungen der Formel II zu Aminoverbindungen der Formel III in Gegenwart von Raneynickel in Alkoholen mit Wasserstoffüberdruck durchführt.

4. Verfahren gemäß Anspruch 1, dadurch gekennzeichnet, daß man den ersten Reaktionsschritt bei Temperaturen zwischen 0 und 50 °C un den zweiten Reaktionsschritt bei Temperaturen zwischen 10 und 50 °C durchführt.

5. Verfahren zur Herstellung eines Arzneimittels enthaltend als Wirkstoff 4-Aminoaryldihydropyridine der allgemeinen Formel (I) gemäß Anspruch 1, dadurch gekennzeichnet, daß man die Wirkstoffe gegebenenfalls unter Verwendung von Hilfs- und Trägerstoffe in üblicher Weise in eine Applikationsform überführt.

## Claims

1. 4-Aminoaryldihydropyridines of the general formula (I)

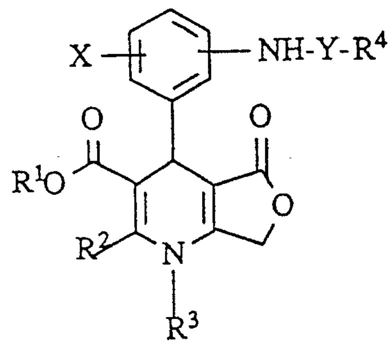

(I)

in which $R^1$ represents a straight-chain, branched or cyclic aliphatic hydrocarbon radical which has up to 10 C atoms and is optionally substituted by $C_1$-$C_6$-alkoxy, $C_1$-$C_6$-alkylsulphonyl, one or more fluorine, chlorine, bromine, cyano or hydroxyl, by a group of the formula

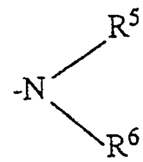

in which

$R^5$ and $R^6$ are identical or different and represent hydrogen, represent $C_1$-$C_6$-alkyl, represent phenyl or benzyl, or represent acetyl or benzoyl, or by phenyl, pyridyl, thienyl, furyl, pyrimidyl, quinolyl or isoquinolyl, it being possible for the phenyl and heteroaryl radicals to carry up to three identical or different substituents from the series comprising fluorine, chlorine, bromine, $C_1$-$C_4$-alkyl, $C_1$-$C_4$-alkoxy, cyano, nitro, di-$C_1$-$C_4$-alkylamino or trifluoromethyl,

$R^2$ represents hydrogen, $C_1$-$C_5$-alkyl or -CN,

$R^3$ represents hydrogen or represents straight-chain or branched $C_1$-$C_4$-alkyl,

X represents hydrogen or represents fluorine, chlorine or bromine,

Y represents the group

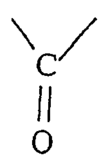

or $SO_2$, and

$R^4$ represents a straight-chain, branched or cyclic, saturated or unsaturated, hydrocarbon radical which has up to 15 carbon atoms and is optionally substituted by one or more fluorine or chlorine, or represents phenyl which is optionally substituted once to three times, identically or differently, by fluorine, chlorine, bromine, $C_1$-$C_6$-alkyl, $C_1$-$C_6$-alkoxy, $C_1$-$C_4$-halogenoalkyl having up to 5 halogen atoms, nitro, cyano, $C_1$-$C_4$-alkylsulphonyl or by a group of the formula

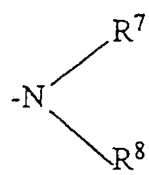

in which
$R^7$ and $R^8$ have the meaning indicated for $R^5$ and
$R^6$ and are identical to or different from the latter,
or represents benzyl or phenethyl, or represents pyridyl, thienyl, furyl, quinolyl or pyrimidyl, each of which is optionally substituted by fluorine, chlorine, $C_1$-$C_4$-alkyl, $C_1$-$C_4$-alkoxy, cyano, nitro or dimethylamino, or represents the group

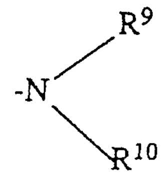

$R^9$ and $R^{10}$ having the meaning indicated for
$R^5$ and $R^6$ and possibly being identical to or different from the latter,
or the group -Y-$R^4$ denotes hydrogen,
in the form of their isomers, isomer mixtures, racemates or optical antipodes and in the form of their physiologically acceptable salts.

**2.** 4-Aminoaryldihydropyridines of the general formula (I) in Claim 1, in which
$R^1$ represents a straight-chain, branched or cyclic aliphatic hydrocarbon radical which has up to 6 C atoms and is optionally substituted by $C_1$-$C_3$-alkoxy, fluorine, chlorine, cyano or hydroxyl, by a group of the formula

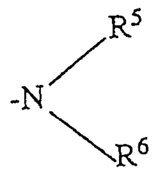

in which
$R^5$ and $R^6$ are identical or different and represent hydrogen, represent $C_1$-$C_4$-alkyl, represent phenyl or benzyl, or represent acetyl,
or by phenyl, pyridyl, pyrimidyl or quinolyl, it being possible for the phenyl and the heteroaryl radicals to be substituted by fluorine, chlorine, methyl, methoxy, cyano, nitro or trifluoromethyl,
$R^2$ represents hydrogen, $C_1$-$C_4$-alkyl or -CN,
$R^3$ represents hydrogen,
X represents hydrogen or represents fluorine,
Y represents the group

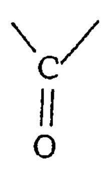

or $-SO_2$, and
$R^4$ represents a straight-chain, branched or cyclic, saturated or unsaturated, hydrocarbon radical which has up to 10 carbon atoms and is optionally substituted by one or more fluorine or chlorine, or represents phenyl which is optionally substituted up to twice, identically or differently, by fluorine, chlorine, $C_1$-$C_4$-alkyl, $C_1$-$C_4$-alkoxy, $C_1$-$C_4$-halogenoalkyl, nitro, cyano or by di-$C_1$-$C_4$-alkylamino, or represents benzyl, or represents pyridyl, furyl, thienyl or quinolyl, each of which is optionally substituted by fluorine, chlorine, methyl, methoxy or nitro, or represents the group of the formula

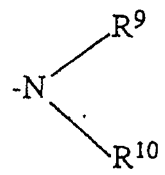

$R^9$ and $R^{10}$ having the meaning indicated for $R^5$ and $R^6$ and being identical to or different from the latter, or the group -Y-$R^4$ denotes hydrogen, in the form of their isomers, isomer mixtures, racemates or optical antipodes and in the form of their physiologically acceptable salts.

3. 4-Aminoaryldihydropyridine according to Claims 1-2 for combating diseases.

4. Medicament containing as active compound 4-aminoaryldihydropyridines according to Claims 1-2.

5. Process for the preparation of 4-aminoaryldihydropyridines of the general formula (I)

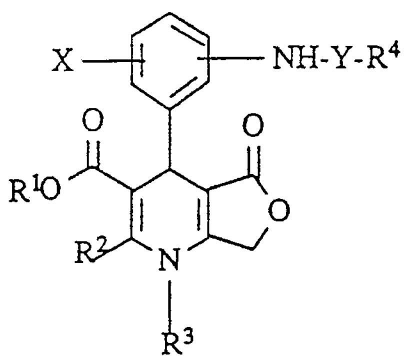

(I)

in which $R^1$, $R^2$, $R^3$, $R^4$, X and Y have the meanings stated in Claim 1, characterized in that nitro compounds of the formula II

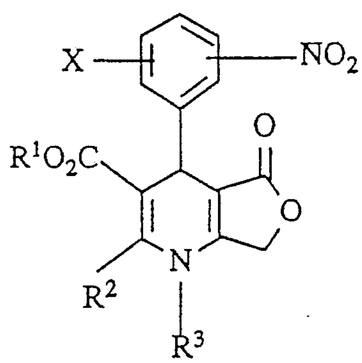

(II)

in which $R^1$, $R^2$, $R^3$, and X have the stated meaning, are hydrogenated by hydrogenation with metal catalysts in the presence of acids at temperatures between -20° C and +100° C in the presence of water or organic solvents to give amino compounds of the general formula III

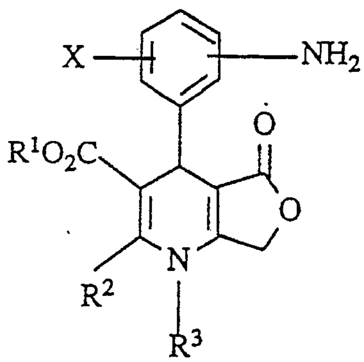

(III)

in which

$R^1$, $R^2$, $R^3$ and X have the stated meaning, and, where appropriate in a second step, the compounds of the formula III are reacted with compounds of the general formula IV

$$R^4 - Y - Z \qquad (IV)$$

in which

$R^4$ and Y have the stated meaning, and

Z represents halogen or represents a group -O-Y-$R^4$ in the presence of inert organic solvents and organic or inorganic bases at temperatures between 0 and 100 °C.

**6.** Process according to Claim 5, characterized in that the reduction of the nitro compounds of the formula II to give amino compounds of the formula III is carried out in the presence of Raney nickel in alcohols with an excess pressure of hydrogen.

**7.** Process according to Claim 5, characterized in that in that the first reaction step is carried out at temperatures between 0 and 50 °C and the second reaction step is carried out at temperatures between 10 and 50 °C.

**8.** Process for the preparation of a medicament according to Claim 4, characterized in that compounds according to Claim 1 are converted in a conventional manner into a form for administration, where appropriate with the use of auxiliaries and vehicles.

**9.** Use of compounds of the general formula I according to Claim 1 for the preparation of medicaments having cardiotonic action, for raising blood pressure, for lowering blood sugar, for reducing mucosal swelling and for influencing the salt and fluid balance.

Claim for the following Contracting State: ES

**1.** Process for the preparation of 4-aminoaryldihydropyridines of the general formula (I)

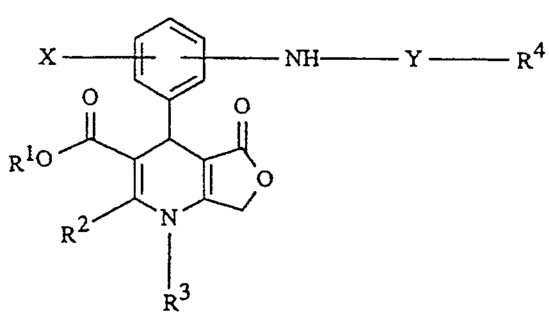

(I)

in which
$R^1$ represents a straight-chain, branched or cyclic aliphatic hydrocarbon radical which has up to 10 C atoms and is optionally substituted by $C_1$-$C_6$-alkoxy, $C_1$-$C_6$-alkylsulphonyl, one or more fluorine, chlorine, bromine, cyano or hydroxyl, by a group of the formula

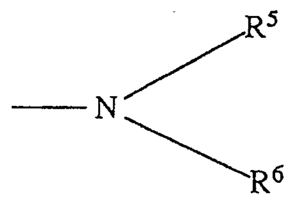

in which
$R^5$ and $R^6$ are identical or different and represent hydrogen, represent $C_1$-$C_6$-alkyl, represent phenyl or benzyl, or represent acetyl or benzoyl, or by phenyl, pyridyl, thienyl, furyl, pyrimidyl, quinolyl or isoquinolyl, it being possible for the phenyl and heteroaryl radicals to carry up to three identical or different substituents from the series comprising fluorine, chlorine, bromine, $C_1$-$C_4$-alkyl, $C_1$-$C_4$-alkoxy, cyano, nitro, di-$C_1$-$C_4$-alkylamino or trifluoromethyl,
$R^2$ represents hydrogen, $C_1$-$C_5$-alkyl or -CN,
$R^3$ represents hydrogen or represents straight-chain or branched $C_1$-$C_4$-alkyl,
X represents hydrogen or represents fluorine, chlorine or bromine,
Y represents the group

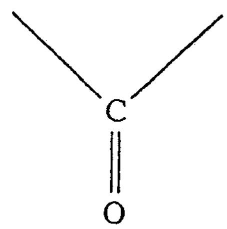

or $-SO_2$, and
$R^4$ represents a straight-chain, branched or cyclic, saturated or unsaturated, hydrocarbon radical which has up to 15 carbon atoms and is optionally substituted by one or more fluorine or chlorine, or represents phenyl which is optionally substituted once to three times, identically or differently, by fluorine, chlorine, bromine, $C_1$-$C_6$-alkyl, $C_1$-$C_6$-alkoxy, $C_1$-$C_4$-halogenoalkyl having up to 5 halogen atoms, nitro, cyano, $C_1$-$C_4$-alkylsulphonyl or by a group of the formula

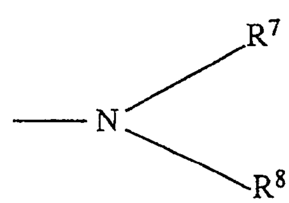

in which
$R^7$ and $R^8$ have the meaning indicated for $R^5$ and
$R^6$ and are identical to or different from the latter,
or represents benzyl or phenethyl, or represents pyridyl, thienyl, furyl, quinolyl or pyrimidyl, each of which is optionally substituted by fluorine, chlorine, $C_1$-$C_4$-alkyl, $C_1$-$C_4$-alkoxy, cyano, nitro or dimethylamino, or represents the group

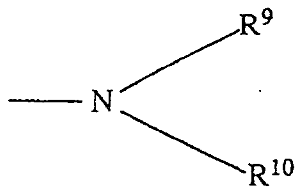

$R^9$ and $R^{10}$ having the meaning indicated for $R^5$ and $R^6$ and possibly being identical to or different from the latter, or the group -Y-$R^4$ denotes hydrogen, in the form of their isomers, isomer mixtures, racemates or optical antipodes and in the form of their physiologically acceptable salts, characterized in that nitro compounds of the general formula (II)

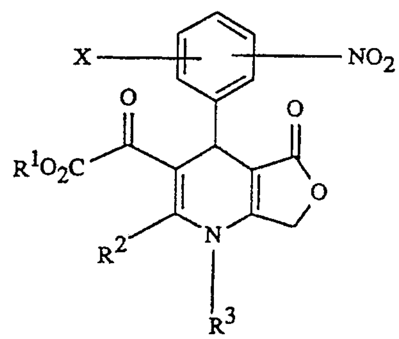

(II)

in which $R^1$, $R^2$, $R^3$ and X have the stated meaning, are hydrogenated by hydrogenation with metal catalysts in the presence of acids at temperatures between -20° C and +100° C in the presence of water or organic solvents to give amino compounds of the general formula (III)

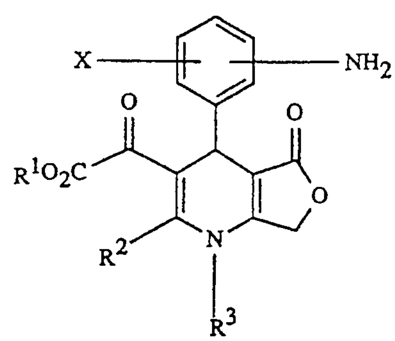

(III)

in which $R^1$, $R^2$, $R^3$ and X have the stated meaning, and, where appropriate in a second step, the compounds of the formula III are reacted with compounds of the general formula IV

$$R^4 - Y - Z \qquad (IV)$$

in which $R^4$ and Y have the stated meaning, and Z represents halogen or represents a group -O-Y-$R^4$ in the presence of inert organic solvents and organic or inorganic bases at temperatures between 0 and 100° C.

2. Process according to Claim 1 for the preparation of 4-aminoaryldihydropyridines of the general formula

(I) according to Claim 1 in which
$R^1$ represents a straight-chain, branched or cyclic aliphatic hydrocarbon radical which has up to 6 C atoms and is optionally substituted by $C_1$-$C_3$-alkoxy, fluorine, chlorine, cyano or hydroxyl, by a group of the formula

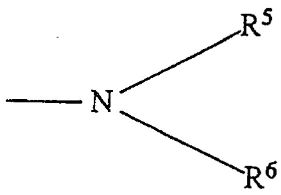

in which
$R^5$ and $R^6$ are identical or different and represent hydrogen, represent $C_1$-$C_4$-alkyl, represent phenyl or benzyl, or represent acetyl, or by phenyl, pyridyl, pyrimidyl or quinolyl, it being possible for the phenyl and the heteroaryl radicals to be substituted by fluorine, chlorine, methyl, methoxy, cyano, nitro or trifluoromethyl,
$R^2$ represents hydrogen, $C_1$-$C_4$-alkyl or -CN,
$R^3$ represents hydrogen,
X represents hydrogen or represents fluorine,
Y represents the group

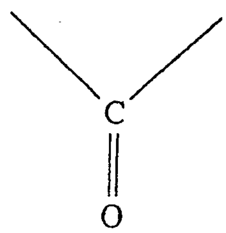

or $-SO_2$, and
$R^4$ represents a straight-chain, branched or cyclic, saturated or unsaturated, hydrocarbon radical which has up to 10 carbon atoms and is optionally substituted by one or more fluorine or chlorine, or represents phenyl which is optionally substituted up to twice, identically or differently, by fluorine, chlorine, $C_1$-$C_4$-alkyl, $C_1$-$C_4$-alkoxy, $C_1$-$C_4$-halogenoalkyl, nitro, cyano or by di-$C_1$-$C_4$-alkylamino, or represents benzyl, or represents pyridyl, furyl, thienyl or quinolyl, each of which is optionally substituted by fluorine, chlorine, methyl, methoxy or nitro, or represents the group of the formula

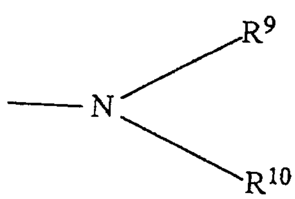

$R^9$ and $R^{10}$ having the meaning indicated for $R^5$ and $R^6$ and being identical to or different from the latter, or the group -Y-$R^4$ denotes hydrogen.

3. Process according to Claim 1, characterized in that the reduction of the nitro compounds of the formula II to give amino compounds of the formula III is carried out in the presence of Raney nickel in alcohols with an excess pressure of hydrogen.

4. Process according to Claim 1, characterized in that the first reaction step is carried out at temperatures

between 0 and 50 °C and the second reaction step is carried out at temperatures between 10 and 50 °C.

5. Process for the preparation of a medicament containing 4-aminoaryldihydropyridines of the general formula (I) according to Claim 1 as active compounds, characterized in that the active compounds are converted in a conventional manner into a form for administration, where appropriate with the use of auxiliaries and vehicles.

## Revendications

1. 4-aminoaryldihydropyridines de formule générale I :

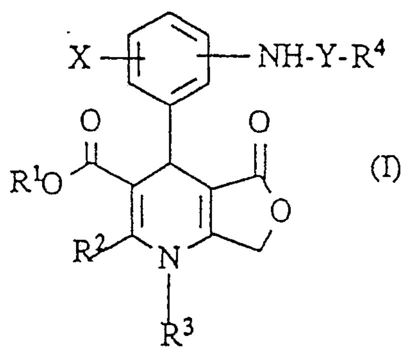

(I)

dans laquelle
$R^1$ représente un radical hydrocarboné aliphatique à chaîne droite, ramifiée ou cyclique contenant jusqu'à 10 atomes de carbone qui peut le cas échéant être substitué par des groupes alcoxy en $C_1$-$C_6$, alkylsulfonyle en $C_1$-$C_6$, un ou plusieurs atomes de fluor, de chlore, de brome ou groupes cyano, hydroxy, par un groupe de formule :

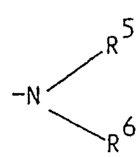

dans laquelle
$R^5$ et $R^6$, ayant des significations identiques ou différentes, représentent chacun l'hydrogène, un groupe alkyle en $C_1$-$C_6$, un groupe phényle ou benzyle, un groupe acétyle ou benzoyle, ou par un groupe phényle, pyridyle, thiényle, furyle, pyrimidyle, quinoléyle ou isoquinoléyle, les groupes phényle et hétéroaryle pouvant porter jusqu'à trois substituants identiques ou différents choisis parmi le fluor, le chlore, le brome, les groupes alkyle en $C_1$-$C_4$, alcoxy en $C_1$-$C_4$, cyano, nitro, di-(alkyle en $C_1$ -$C_4$ )-amino ou trifluorométhyle,
$R^2$ représente l'hydrogène, un groupe alkyle en $c_1$-$C_5$, - CN,
$R^3$ représente l'hydrogène ou un groupe alkyle à chaîne droite ou ramifiée en $C_1$-$C_4$,
X représente l'hydrogène ou le fluor, le chlore ou le brome,
Y représente un groupe

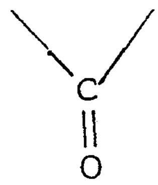

ou $-SO_2$, et

$R^4$ représente un radical hydrocarboné saturé ou insaturé à chaîne droite, ramifiée ou cyclique, contenant jusqu'à 15 atomes de carbone qui peut le cas échéant être substitué par un ou plusieurs atomes de fluor ou de chlore, ou bien

$R^4$ représente un groupe phényle portant le cas échéant un à trois substituants identiques ou différents choisis parmi le fluor, le chlore, le brome, les groupes alkyle en $C_1$-$C_6$, alcoxy en $C_1$-$C_6$, halogénoalkyle en $C_1$-$C_4$ contenant jusqu'à 5 atomes d'halogènes, nitro, cyano, alkylsulfonyle en $C_1$ -$C_4$, ou par un groupe de formule :

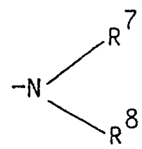

dans laquelle

$R^7$, $R^8$ ont les significations indiquées pour $R^5$, $R^6$ mais sont identiques à ceux-ci ou différents de ceux-ci,

ou bien

$R^4$ représente un groupe benzyle ou phénéthyle, ou bien un groupe pyridyle, thiényle, furyle, quinoléyle ou pyrimidyle éventuellement substitué par le fluor, le chlore, des groupes alkyle en $C_1$-$C_4$, alcoxy en $C_1$-$C_4$, cyano, nitro ou diméthylamino, ou bien le groupe :

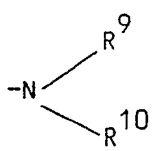

dans lequel

$R^9$, $R^{10}$ ont les significations indiquées pour $R^5$, $R^6$ mais sont identiques à ceux-ci ou différents de ceux-ci,

ou bien -Y-$R^4$ représente l'hydrogène,

à l'état d'isomères, de mélanges d'isomères, de racémates, d'antipodes optiques et également à l'état de sels acceptables pour l'usage pharmaceutique.

**2.** 4-aminoaryldihydropyridines de formule générale I de la revendication 1, dans laquelle

$R^1$ représente un radical hydrocarboné aliphatique à chaîne droite, ramifiée ou cyclique contenant jusqu'à 6 atomes de carbone et éventuellement substitué par des groupes alcoxy en $C_1$-$C_3$, le fluor, le chlore, des groupes cyano, hydroxy, par un groupe de formule :

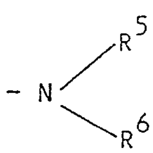

dans laquelle
$R^5$, $R^6$ , ayant des significations identiques ou différentes, représentent chacun l'hydrogène, un groupe alkyle en $C_1$-$C_4$, un groupe phényle ou benzyle, un groupe acétyle,
ou par des groupes phényle, pyridyle, pyrimidyle ou quinoléyle, les groupes phényle et hétéroaryle pouvant être substitués par le fluor, le chlore, des groupes méthyle, méthoxy, cyano, nitro ou trifluorométhyle,
$R^2$ représente l'hydrogène, un groupe alkyle en $C_1$-$C_4$, -CN,
$R^3$ représente l'hydrogène,
X représente l'hydrogène ou le fluor,
Y représente le groupe

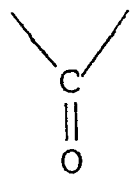

ou $-SO_2$, et
$R^4$ représente un radical hydrocarboné saturé ou insaturé à chaîne droite, ramifiée ou cyclique contenant jusqu'à 10 atomes de carbone et éventuellement substitué par un ou plusieurs atomes de fluor ou de chlore, ou bien
$R^4$ représente un groupe phényle portant éventuellement jusqu'à deux substituants identiques ou différents choisis parmi le fluor, le chlore, les groupes alkyle en $C_1$-$C_4$, alcoxy en $C_1$-$C_4$, halogénoalkyle en $C_1$-$C_4$, nitro, cyano ou di-(alkyle en $C_1$ -$C_4$)-amino, ou bien
$R^4$ représente un groupe benzyle, ou bien
$R^4$ représente un groupe pyridyle, furyle, thiényle ou quinoléyle éventuellement substitué par le fluor, le chlore, des groupes méthyle, méthoxy ou nitro, ou bien
$R^4$ représente le groupe de formule :

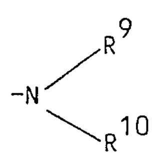

dans laquelle
$R^9$, $R^{10}$ ont les significations indiquées pour $R^5$, $R^6$ mais sont identiques à ceux-ci ou différents de ceux-ci,
ou bien $-Y-R^4$ représente l'hydrogène,
à l'état d'isomères, de mélanges d'isomères, de racémates, d'antipodes optiques et également à l'état de sels acceptables pour l'usage pharmaceutique.

3. Une 4-aminoaryldihydropyridine selon la revendication 1 ou 2, pour le traitement de maladies.

4. Médicaments contenant en tant que substance active une 4-aminoaryldihydropyridine selon les revendications 1 et 2.

5. Procédé de préparation des 4-aminoaryldihydropyridines de formule générale :

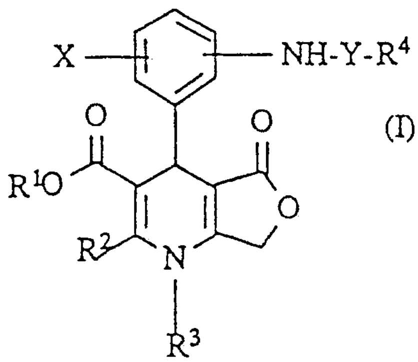

(I)

dans laquelle
$R^1$, $R^2$, $R^3$, $R^4$, X et Y ont les significations indiquées dans la revendication 1, caractérisé en ce que l'on réduit des dérivés nitrés de formule II :

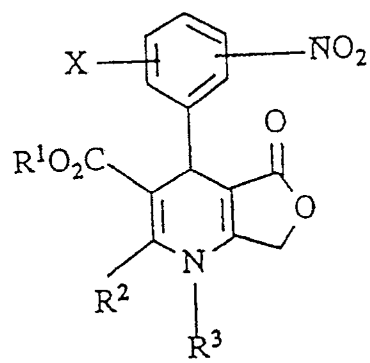

(II)

dans laquelle
$R^1$, $R^2$, $R^3$ et X ont les significations indiquées ci-dessus, en les dérivés aminés de formule générale III :

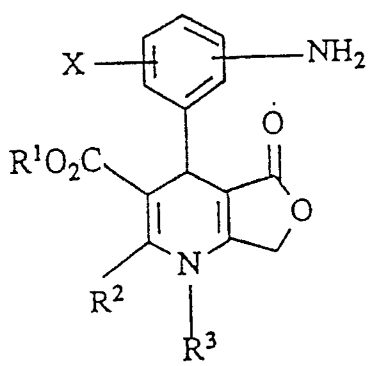

(III)

dans laquelle
$R^1$, $R^2$, $R^3$ et X ont les significations indiquées ci-dessus, par hydrogénation sur des catalyseurs métalliques en présence d'acides à des températures allant de -20 à +100° C, en présence d'eau ou de solvants organiques, et, le cas échéant, dans un deuxième stade opératoire, on fait réagir les composés de formule III avec des composés de formule IV :

$R^4$-Y-Z IV

dans laquelle
$R^4$ et Y ont les significations indiquées ci-dessus, et
Z représente un halogène ou un groupe -O-Y-$R^4$, en présence de solvants organiques inertes et de bases organiques ou minérales, à des températures allant de 0 à 100 °C.

6. Procédé selon la revendication 5, caractérisé en ce que l'on réduit les dérivés nitrés de formule II en dérivés aminés de formule III en présence de nickel de Raney dans des alcools par l'hydrogène sous pression.

7. Procédé selon la revendication 5, caractérisé en ce que le premier stade de réaction est réalisé à des températures de 0 à 50 °C et le second à des températures de 10 à 50 °C.

8. Procédé de préparation d'un médicament selon la revendication 4, caractérisé en ce que l'on met des composés de la revendication 1 sous une forme d'administration, de la manière habituelle, par utilisation de produits auxiliaires et de véhicules.

9. Utilisation des composés de formule générale 1 selon la revendication 1, pour la préparation de médicaments à activité cardiotonique servant à accroître la pression sanguine, à diminuer la teneur en sucre du sang, à désenfler les muqueuses et à intervenir dans le métabolisme des sels et des liquides.

Revendications pour l'Etat Contractant Suivant: Espagne.

1. Procédé de préparation des 4-aminoaryldihydropyridines de formule générale I :

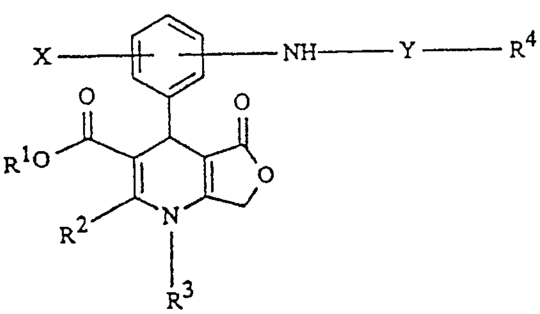
(I)

dans laquelle

$R^1$ représente un radical hydrocarboné aliphatique à chaîne droite, ramifiée ou cyclique contenant jusqu'à 10 atomes de carbone et éventuellement substitué par des groupes alcoxy en $C_1$-$C_6$, alkylsulfonyle en $C_1$-$C_6$, un ou plusieurs atomes de fluor, de chlore, de brome ou groupes cyano, hydroxy, par un groupe de formule :

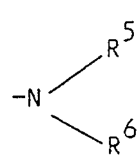

dans laquelle
$R^5$ et $R^6$, ayant des significations identiques ou différentes, représentent chacun l'hydrogène, un groupe alkyle en $C_1$-$C_6$, un groupe phényle ou benzyle, un groupe acétyle ou benzoyle,

ou par des groupes phényle, pyridyle, thiényle, furyle, pyrimidyle, quinoléyle ou isoquinoléyle, les groupes phényle et hétéroaryle pouvant porter jusqu'à trois substituants identiques ou différents choisis parmi le fluor, le chlore, le brome, les groupes alkyle en $C_1$-$C_4$, alcoxy en $C_1$-$C_4$, cyano, nitro, di-(alkyle en $C_1$ -$C_4$)-amino ou trifluorométhyle,

$R^2$ représente l'hydrogène, un groupe alkyle en $C_1$-$C_5$ , -CN,

$R^3$ représente l'hydrogène ou un groupe alkyle à chaîne droite ou ramifiée en $C_1$-$C_4$,

X représente l'hydrogène ou le fluor, le chlore ou le brome,

Y représente le groupe

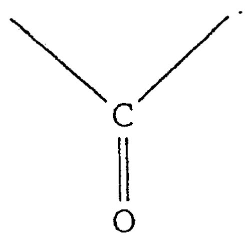

ou le groupe -$SO_2$, et

$R^4$ représente un radical hydrocarboné saturé ou insaturé à chaîne droite, ramifiée ou cyclique, contenant jusqu'à 15 atomes de carbone et éventuellement substitué par un ou plusieurs atomes de fluor ou de chlore, ou bien

$R^4$ représente un groupe phényle portant éventuellement un à trois substituants identiques ou différents choisis parmi le fluor, le chlore, le brome, les groupes alkyle en $C_1$ -$C_6$, alcoxy en $C_1$-$C_6$, halogénoalkyle en $C_1$-$C_4$ contenant jusqu'à 5 atomes d'halogènes, nitro, cyano, alkylsulfonyle en $C_1$-$C_4$, ou par un groupe de formule :

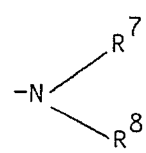

dans laquelle

$R^7$, $R^8$ ont les significations indiquées pour $R^5$, $R^6$ mais sont identiques à ceux-ci ou différents de ceux-ci, ou bien

$R^4$ représente un groupe benzyle ou phénéthyle, ou bien un groupe pyridyle, thiényle, furyle, quinoléyle ou pyrimidyle éventuellement substitué par le fluor, le chlore, des groupes alkyle en $C_1$ -$C_4$, alcoxy en $C_1$-$C_4$, cyano, nitro ou diméthylamino, ou bien le groupe :

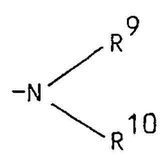

dans lequel

$R^9$, $R^{10}$ ont les significations indiquées pour $R^5$, $R^6$ mais sont identiques à ceux-ci ou différents de ceux-ci,

ou bien -Y-$R^4$ représente l'hydrogène,

à l'état d'isomères, de mélanges d'isomères, de racémates, d'antipodes optiques et également à l'état de sels acceptables pour l'usage pharmaceutique,

caractérisé en ce que l'on réduit des dérivés nitrés de formule générale II :

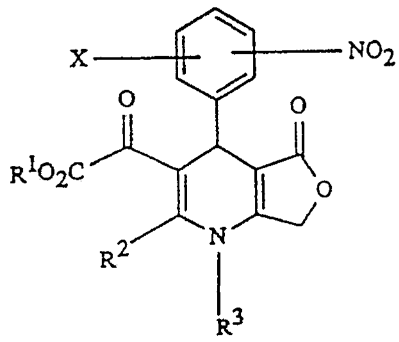

(II)

dans laquelle
$R^1$, $R^2$, $R^3$ et X ont les significations indiquées ci-dessus, en dérivés aminés de formule générale III :

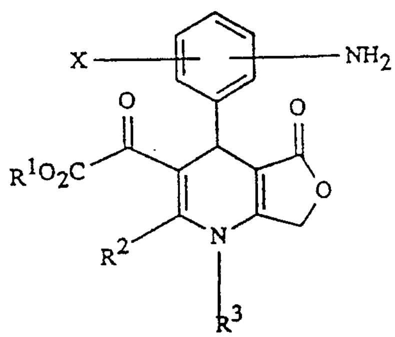

(III)

dans laquelle
$R^1$, $R^2$, $R^3$ et X ont les signification indiquées ci-dessus, par hydrogénation sur des catalyseurs métalliques en présence d'acides à des températures allant de -20 à +100° C, en présence d'eau ou de solvants organiques, et, le cas échéant, dans un deuxième stade opératoire, on fait réagir les composés de formule III avec des composés de formule générale IV :

$$R^4 —— Y —— Z \qquad IV$$

dans laquelle
$R^4$, Y ont les significations indiquées ci-dessus, et
Z représente un halogène ou le groupe -O-Y-$R^4$,
en présence de solvants organiques inertes et de bases organiques ou minérales, à des températures de 0 à 100° C.

**2.** Procédé selon la revendication 1, pour préparer les 4-amiroaryldihydropyridines de formule générale I de la revendication 1 dans laquelle
$R^1$ représente un radical hydrocarboné aliphatique à chaîne droite, ramifiée ou cyclique contenant jusqu'à 6 atomes de carbone et éventuellement substitué par des groupes alcoxy en $C_1$-$C_3$, le fluor, le chlore, des groupes cyano, hydroxy, par un groupe de formule :

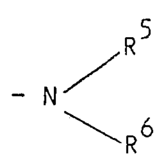

dans laquelle
$R^5$ et $R^6$ , ayant des significations identiques ou différentes, représentent chacun l'hydrogène, un groupe alkyle en $C_1$-$C_4$, un groupe phényle ou benzyle, un groupe acétyle,
ou par un groupe phényle, pyridyle, pyrimidyle ou quinoléyle, les groupes phényle et hétéroaryle pouvant être substitués par le fluor, le chlore, des groupes méthyle, méthoxy, cyano, nitro ou trifluorométhyle,
$R^2$ représente l'hydrogène, un groupe alkyle en $C_1$ -$C_4$, -CN,
$R^3$ représente l'hydrogène,
X représente l'hydrogène ou le fluor,
Y représente le groupe

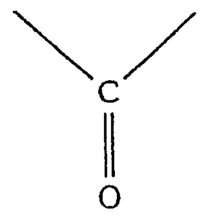

ou -$SO_2$, et $R^4$ représente un radical hydrocarboné saturé ou insaturé à chaîne droite, ramifiée ou cyclique contenant jusqu'à 10 atomes de carbone, et éventuellement substitué par un ou plusieurs atomes de fluor ou de chlore, ou bien
$R^4$ représente un groupe phényle portant éventuellement jusqu'à deux substituants identiques ou différents choisis parmi le fluor, le chlore, les groupes alkyle en $C_1$ -$C_4$, alcoxy en $C_1$-$C_4$, halogénoalkyle en $C_1$-$C_4$, nitro, cyano ou di-(alkyle en $C_1$ -$C_4$)-amino, ou bien
$R^4$ représente un groupe benzyle, ou bien
$R^4$ représente un groupe pyridyle, furyle, thiényle ou quinoléyle éventuellement substitué par le fluor, le chlore, des groupes méthyle, méthoxy ou nitro, ou bien
$R^4$ représente le groupe de formule :

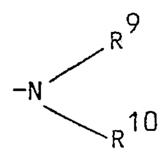

dans laquelle
$R^9$ $R^{10}$ ont les significations indiquées pour $R^5$ , $R^6$ mais sont identiques à ceux-ci ou différents de ceux-ci, ou bien -Y-$R^4$ représente l'hydrogène.

3. Procédé selon la revendication 1, caractérisé en ce que l'on réduit les dérivés nitrés de formule II en dérivés aminés de formule III en présence de nickel de Raney, dans des alcools, par l'hydrogène sous pression.

4. Procédé selon la revendication 1, caractérisé en ce que le premier stade de réaction est réalisé à des températures de 0 à 50 °C et le deuxième à des températures de 10 à 50 °C.

5. Procédé de préparation d'un médicament contenant en tant que substance active une 4-aminoaryldihydropyridine de formule générale I de la revendication 1, caractérisé en ce que l'on met la substance active de la manière habituelle sous une forme d'administration, éventuellement en utilisant des produits auxiliaires et des véhicules.